Europäisches Patentamt

(19) European Patent Office

Office européen des brevets

(11) Publication number: **0 030 014**
**B1**

(12) **EUROPEAN PATENT SPECIFICATION**

(45) Date of publication of patent specification: **12.03.86**

(21) Application number: **80107427.9**

(22) Date of filing: **27.11.80**

(51) Int. Cl.⁴: **A 61 B 1/26,** A 61 B 1/06

(54) **A combination of a laryngoscope and a flexible tube.**

(30) Priority: **28.11.79 US 98271**
**28.04.80 US 144704**

(43) Date of publication of application:
**10.06.81 Bulletin 81/23**

(45) Publication of the grant of the patent:
**12.03.86 Bulletin 86/11**

(84) Designated Contracting States:
**CH DE FR GB LI NL**

(56) References cited:
**DE-A-1 566 116**
**DE-A-2 738 202**
**FR-A-2 361 855**
**GB-A-1 546 000**
**US-A-3 638 644**
**US-A-3 826 248**
**US-A-3 908 665**
**US-A-3 913 568**

(73) Proprietor: **Upsher, Michael S.**
**2957 Adeline Drive**
**Burlingame California 94010 (US)**

(72) Inventor: **Upsher, Michael S.**
**2957 Adeline Drive**
**Burlingame California 94010 (US)**

(74) Representative: **Dipl.-Phys.Dr. Manitz Dipl.-Ing.**
**Finsterwald Dipl.-Ing. Grämkow Dipl.-Chem.Dr.**
**Heyn Dipl.-Phys.Rotermund**
**B.Sc. Morgan Robert-Koch-Strasse 1**
**D-8000 München 22 (DE)**

Courier Press, Leamington Spa, England.

## Description

The present invention relates to a combination of a laryngoscope and a flexible tube, the laryngoscope having a handle, and a blade with a base and a tip, wherein said blade can be coupled by said base to said handle, the blade comprising an elongate member curved for insertion into the throat of a patient and into the pharynx, and the elongate member being shaped to facilitate the introduction of the flexible tube during intubation. A laryngoscope of this kind is described in the introduction to DE—A—27 38 202 with reference to the laryngoscope described in US—A—3 638 644.

In using conventional laryngoscopes, it is only by trial and error that the blade of the laryngoscope can be inserted into the pharynx in such a manner to elevate the epiglottis so that an endotracheal tube can be manually moved into the throat and into the larynx. This is because the larynx cannot always be directly and completely viewed during insertion of the blade. Usually, the laryngoscope is held in one hand as the endotracheal tube is held in the other hand, because the laryngoscope has no means for both removably holding and guiding the endotracheal tube. This is an awkward situation because the user of the laryngoscope must be able to probe the pharynx without actually seeing the epiglottis in an attempt to elevate it, yet the user must be ready to insert the tube as soon as the epiglottis is elevated. Most importantly, the line of sight from the eye of the anesthesiologist to the epiglottis and the larynx must be straight using direct vision; whereas, the endotracheal tube must frequently be passed in a curved manner to conform with the normal anatomical pharyngeal curvature. The anesthesiologist, in these cases, is therefore asked to straighten out the normal physiological curve of the pharynx. This may result in damage to the patient's teeth and "soft parts". Thus, considerable time and effort is expended in elevating the epiglottis and then inserting the tube, all of which must be done while causing only a minimal amount of discomfort to the patient.

The insertion of a conventional laryngoscope in the throat is a very tedious process in many cases and, in some cases, injury is caused to the patient by virtue of the movements of the laryngoscope blade within the narrow confines of the physiological limitations of the patient. No satisfactory laryngoscope has been heretofore provided for effectively guiding the endotracheal tube into the throat while providing for the direct and indirect viewing of the throat during the insertion of the blade to elevate the epiglottis.

Representative laryngoscopes are disclosed in the following references US—A—2,646,036, 3,986,854 and US—A—4,086,919. An endoscope which is related to a laryngoscope is disclosed in US—A—3,896,793. The laryngoscopes of these references do not provide for the guiding of an endotracheal tube while permitting direct and indirect viewing of the throat during insertion of the blade of the laryngoscope. Moreover, there is no teaching or suggestion in these references that the blade can be shaped to fit different physiological throat configurations of various patients, nor do the references suggest the need for improvement in laryngoscope blades.

The principal object of this invention is to provide an improved laryngoscope and flexible tube combination which permits both direct and indirect viewing of the pharynx and larynx as the blade of the laryngoscope is inserted into the pharynx, and which permits effective guidance of the endotracheal tube into the throat to thereby eliminate a trial and error method of elevating the epiglottis and guiding the endotracheal tube into position.

Another object of the present invention is to provide a combination of the type described which permits shaping of the blade to accommodate different throat configurations of various patients without destroying the capability of the blade to guide the endotracheal tube into the larynx past the epiglottis after the latter has been elevated by the blade.

Another object of the present invention is to provide an improved combination of the type described, wherein the laryngoscope has a light source and wherein the elongate member can either be integral with the base or removably mounted on the base to permit the elongate member to be thrown away after a single use.

In order to satisfy the principal object enunciated above the present invention provides a combination of the initially named kind which is characterised in that said blade is tubular with a passage for the introduction of said flexible tube; and in that a slot in communication with said passage extends at least partway along the length of said passage, wherein said slot is narrower than said passage and narrow enough to hold the tube in the passage but wide enough to allow said flexible tube to be separated from the blade when the tube yields so as to become elliptical in shape sufficiently to squeeze or pass through the slot.

If the laryngoscope is of the operating type, the slot does not extend throughout the length of the blade but extends only a short distance from the outer end of the blade.

The slot thus not only improves visibility but also permits separation of the blade from the endotracheal tube after the tube has been inserted into the trachea.

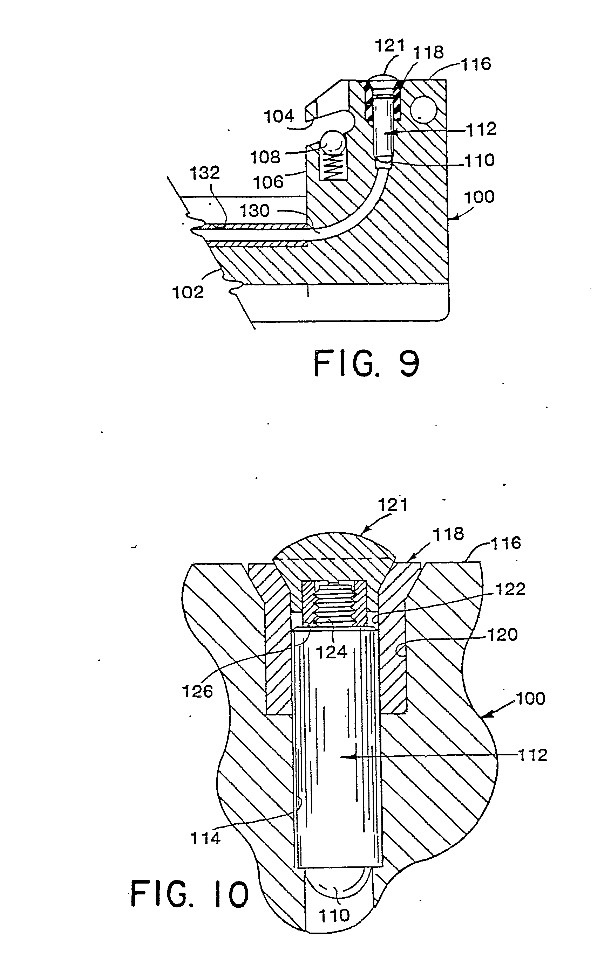

An oro-pharyngeal airway comprising an extended elongated body portion formed in the general shape of the normal pharyngeal contour, with a tubular passageway of rectangular cross-section and a slot extending in communication with said passage extending along said body portion, is admittedly known from Fig. 9 of US—A—3 908,665. The slot of Fig. 9 of US—A—3 908 665 is however not provided for intubation but rather to permit visibility of the passageway defined to permit cleaning.

Other improvements of the laryngoscope of the present invention include the fact that the viewing slot, instead of being on the margin of the blade, can be located at a position within an arc of 90° on each side of the back margin of the blade. This feature provides greater length of insertion of the blade before direct viewing is blocked due to the curvature of the blade.

In the preferred embodiment the laryngoscope has a light guide mounted in said elongate member for transmitting light from a light source adjacent said base to a position adjacent said tip, as known from US—A—3 638 644 and is characterised in that the light guide comprises a fibre optic guide which extends through a bore in said elongate member.

This latter characterising feature is admittedly known per se from DE—A—27 38 202 or from GB—A—1 546 000, however not in combination with the tubular blade of the present invention.

In another embodiment a flexible fibre optic viewing guide is provided which extends from an eyepiece movably positioned outside of said blade along said elongate member to said tip. A similar arrangement is admittedly known from GB—A 1 546 000 in the latter case the eyepiece is however secured to the blade.

In one embodiment the elongate member and the base are integral with one another.

In another embodiment, the elongate member and base can be separable from each other. This permits the blade to be disposable and made sufficiently inexpensive to permit it to be thrown away after a single use. To this end, the base has a tubular connector part which releasably receives one end of the elongate member, the latter being of yieldable material so that the end of the elongate member can be snap fitted into place into the connector part of the base. In the alternative the connector part of the base can be yieldable and the end of the blade can be rigid.

With the disposable elongate member a light guide is carried by the base and extends outwardly from the base, along the elongate member and removably through an opening near the tip of the elongate member. This allows the blade to become separated from the light guide as well as the base yet the light guide can transmit light along the blade and to the outer end of the blade. A second light guide for viewing the illuminated area in advance of the blade can be provided as a separate element or can be formed with the first-mentioned light guide as a single unit.

In yet another embodiment the blade can be made of a suitable material which allows the blade to be normally rigid but capable of being flexed or shaped to fit the physiological throat configurations of different patients.

The light source may be provided in an adapter removably mounted on the handle. In this arrangement, wherein the handle has, in manner known per se for coupling of a blade, a first pair of parallel spaced apart walls defining a first slot and a first cross-bar extending across said first slot, and wherein said handle is adapted to receive a battery power source and has a first electrical contact provided adjacent the base of the slot, the laryngoscope is characterised in that an adapter is provided for coupling said blade to said handle, said adapter having a first end connectable with said handle and a second end connectable with said base of said blade; in that said first end has a projection having two spaced side walls adapted to engage in said first slot, a transverse groove adapted to engage with said first cross-bar, and locking means for locking said projection in position in said first slot; in that said second end has a second pair of parallel spaced apart walls defining a second slot and a second cross-bar extending across said second slot; in that said base of said blade has a second projection having two spaced side walls adapted to engage in said second slot and a transverse groove adapted to engage with second cross-bar, there being detent means for releasably securing said base of said blade with said second end of said adapter in an engaged position; in that said handle has a first electrical contact substantially centrally disposed in said first slot facing a cooperating second contact provided on a base portion of said first projection; in that said second contact is electrically connected to a third contact substantially centrally disposed in said second slot; in that said second contact is electrically connected to a light source provided in said adapter; in that a switch is provided in said adapter, said switch being actuatable by said blade in said engaged position to complete an electrical circuit between said light source and said battery power source; in that a second blade having a second light source at its tip can be substituted for the first said blade, said second blade having a fourth contact cooperating with said third contact and a fifth contact cooperating with said second cross-bar to complete an electrical circuit to said battery power source to energise said second light source, said second blade being constructed so as not to actuate said switch means; and in that said first and second cross-bars are electrically connected together and to said battery power source when said adapter is locked in position in said first slot.

With this arrangement the adapter can have a body portion of metal thus ensuring electrical contact between said first and second cross-bars.

In one variant the locking means comprises a pair of wedges mounted in a passage in said first projection and a set screw for forcing said wedges outwardly against said first pair of spaced apart walls.

In the Drawings

Fig. 1 is a fragmentary, cross-sectional view of a portion of an improved laryngoscope blade of the present invention, showing the light source carried by the base integral with the elongate member;

Fig. 2 is a side elevational view of the tubular blade of this invention showing fiber optic light and visual guides in dashed lines extending along the length of the blade;

Fig. 3 is a perspective view of the laryngoscope blade of Fig. 2, looking in the direction of the end of the blade adjacent to the base thereof;

Fig. 4 is a fragmentary, perspective view of the blade looking downwardly from above the blade shown in Fig. 2;

Fig. 5 is a cross-sectional view taken along line 5—5 of Fig. 2;

Fig. 6 is a view similar to Fig. 1 but showing the base and attachment means for a semi-rigid, disposable elongate member separable from the base;

Fig. 7 is a view similar to Fig. 2 but showing the disposable elongate member removed from the base with the elongate member being shown in dashed lines;

Fig. 8 is a side elevational view of the elongate member for releasable attachment to the base of Fig. 7;

Fig. 9 is a view similar to Fig. 1 but showing another base for a laryngoscope blade;

Fig. 10 is an enlarged, fragmentary cross-sectional view of the bulb in the base of Fig. 9;

Figs. 11 and 12 are views similar to Fig. 9 but showing additional ways of mounting the bulb in the base;

Fig. 13 is an enlarged, fragmentary cross-sectional view of a laryngoscope handle having a bulb mounted therein for a standard laryngoscope blade or a blade having a fiberoptics bundle for transmitting light to the outer end of the blade from the light bulb in the handle;

Figs. 13A and 13B show a side elevational view and a top plan view respectively of a cartridge for use in the laryngoscope handle of Fig. 13;

Fig. 14 is a view of the handle of Fig. 13 but rotated through an angle of 90°;

Fig. 15 is a top plan view of the handle of Fig. 13;

Fig. 16 is a view similar to Fig. 13 but showing the bulb and a switch in a cartridge for mounting in a conventional laryngoscope handle;

Fig. 17 is a view of the cartridge of Fig. 16 but rotated through an angle of 90°;

Fig. 18 is an exploded view of the handle showing the cartridge for insertion thereinto;

Fig. 19 is a cross-sectional view of a metallic adapter for mounting in a standard laryngoscope handle;

Fig. 20 is a second cross-sectional view of the adapter in Fig. 19;

Fig. 21 is an enlarged cross-sectional view of the locking mechanism for holding the adapter of Figs. 19 and 20 in place on a handle;

Figs. 22, 23, and 24 are different elevational views of the adapter;

Fig. 25 is a top plan view of the adapter;

Figs. 26 and 27 are views similar to Figs. 19 and 20 but showing a plastic adapter;

Fig. 28 is a side elevational view of either adapter on a conventional handle;

Fig. 29 is a top plan view of the adapter of Fig. 26 and 27;

Fig. 30 is a side elevational view, partly in section, of a handle having a bulb mounted in the upper end thereof for use with a conventional laryngoscope blade;

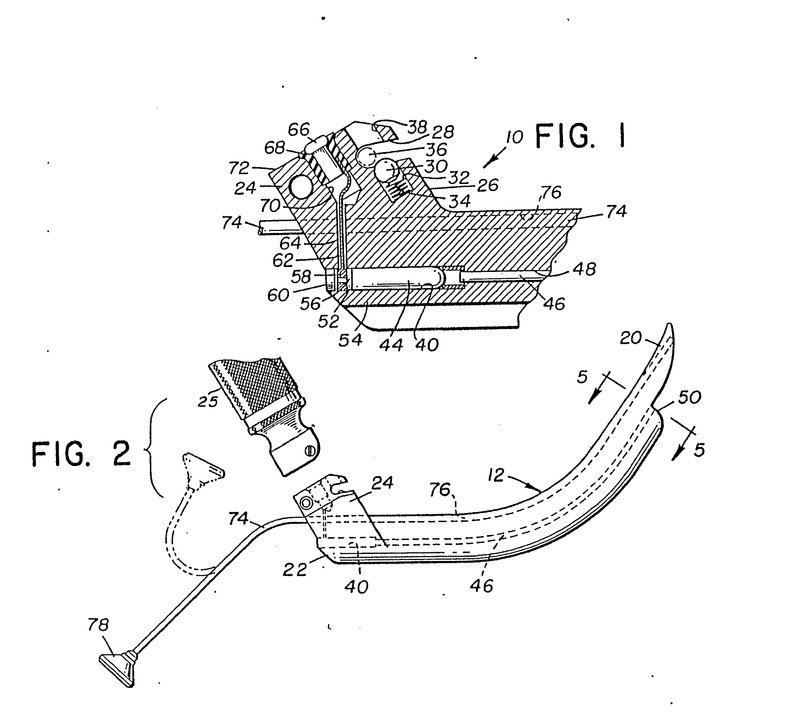

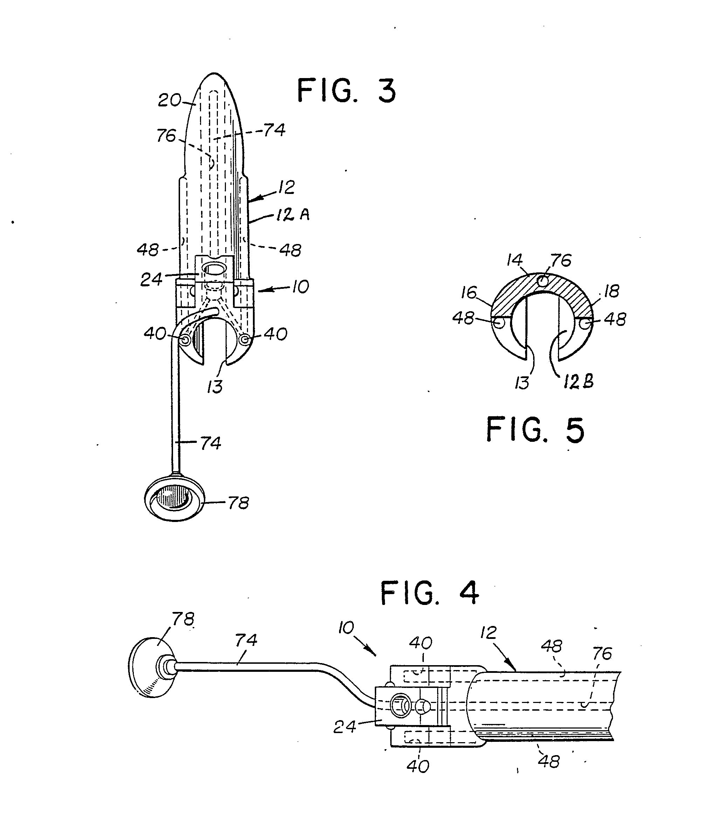

A first embodiment of the laryngoscope of the present inventions is broadly denoted by the numeral 10 and is shown in Figs. 1—4. Unit 10 includes a tubular laryngoscope blade 12 with a base 24 and an elongate curved member 12A which is curved in the manner shown in Fig. 2 and provided with slot 13 along its length in the manner shown in Fig. 5 to present a top portion 14 and a pair of sides 16 and 18. The blade is tubular throughout its entire length except for an end tip 20 which serves to elevate the epiglottis of a patient when the blade is inserted into the pharynx and larynx of the patient. The tubular and slotted nature of the blade permits an endotracheal tube (not shown) to be inserted in an open end (Fig. 2) 22 of blade 12 and to be guided along the blade so that the tube can emerge near tip 20 after the blade has been inserted into the throat of a patient. This allows the tube to enter the patient's trachea for intubation as is well known. The tube is curved to permit it to follow the conformation of the throat and to facilitate insertion of blade 12 into the throat prior to intubation. The blade could also be of a material which is normally rigid but can be flexed or shaped to fit different throat configurations. Slot 13 permits separation of the blade from the endotracheal tube when the latter is in the trachea.

Blade 12 has a base 24 adjacent to end 22 thereof, the base adapted to be coupled in a well-known manner to a conventional laryngoscope handle 25 (Fig. 2) containing a battery 27 for energizing a light source hereinafter described. Base 24 has a surface 26 provided with a groove 28 therein for receiving the cross-bar 36 on the handle. A ball detent 30 in a bore 32 in the base is spring biased into groove 28 by a spring 34 so that the ball releasably holds bar 36 in place in the dashed line position of Fig. 1 to releasably connect the handle to the blade. A bore 38 is formed in base 24 to facilitate the drilling of bore 32.

Blade 12 has a pair of bores 40 (Figs. 1 and 4) at the opposed sides thereof which removably receive respective light bulbs 44, only one of which is shown in Fig. 1. The light bulbs define light sources which are energized by the battery in the handle when the handle is connected to blade 12, and the light from the light source is directed out of bores 40 through fiber optic light guides 46 which extend into and through bores 48 extending along and through the opposed sides 16 and 18 of blade 12 as is shown in Fig. 4 and 5. The light guides terminate at end edges 50 (Fig. 2) of blade 12, and the end faces of light guides 46 define the light sources which project light forwardly of the outer end of the blade and longitudinally of tip 20 to illuminate a patient's throat area in advance of the blade when the blade is being inserted into the throat. While a pair of light sources or light bulbs 44 have been shown for purposes of illustration, it is clear that only one light bulb could be deemed necessary. The fact that light sources are in the base of the blade rather than mounted externally of the blade near edges 50 permits a compact assembly of light source and blade and eliminates electrical contact problems as well as heat that is generated in the vicinity of edges 50 when the light bulb or light source is adjacent to such edges.

Means is provided to electrically connect each light bulb 44 to the battery in the handle when the handle is coupled with the blade. For instance, each light bulb 44 is provided with a base 52 which makes electrical contact with the adjacent metallic portion 54 of base 24, and the bulb has a center terminal 56 engaged by a contact ring 58 electrically insulated from base portion 54 and held in place by an electrically threaded access · cap 60 threaded into bore 40. A conductor wire 62 in electrical contact with contact ring 58 is directed through a respective bore 64 and makes electrical contact with an electrical terminal 66 press-fitted by means of a rubber grommet 68 in a second larger bore 70 extending into the end face 72 of base 24. Terminal 66 is common to the wires 62 of both bulbs 44.

When base 24 is releasably coupled to the handle with cross-bar 36 in the dashed line position of Fig. 1, terminal 66 makes electrical contact with the corresponding center terminal 73 of the battery in the handle. Moreover, bar 36, which is metallic, makes electrical contact with the bottom terminal of the battery and with base 24 so that the electrical circuit through the battery and the bulbs is completed, whereupon the bulbs are energized and the light from the bulbs passes through respective light guides 46 to their end faces near edges 50.

A fiber optic visual guide 74 extends through a bore 76 along the top of blade 12, and this visual guide extends through base 24 and outwardly therefrom. Visual guide 74 has an eyepiece 78 which permits viewing of the illuminated area in advance of the blade. Moreover, the part of light guide 74 extending outwardly from base 24 is flexible so that eyepiece 78 can be placed at various positions relative to the blade for the convenience of the user.

In use, blade 12 is releasably coupled to the handle and this causes the light bulbs to be energized immediately. Then, either while sighting through eyepiece 78 or viewing the pharynx directly through the slot in the blade, the user inserts the blade into the throat of the patient, during which time the throat area is illuminated by the light emanating from light guides 46 and the reflected light can be viewed through directly or through visual guide 74. When the blade is properly inserted in the pharynx and larynx, the endotracheal tube is guided through the tubular portion of blade 12 and into the trachea of the patient, following which the blade can then be separated from the endotracheal tube by causing the tube to pass through slot 13 of the blade while the blade is removed from the throat as the endotracheal tube remains.

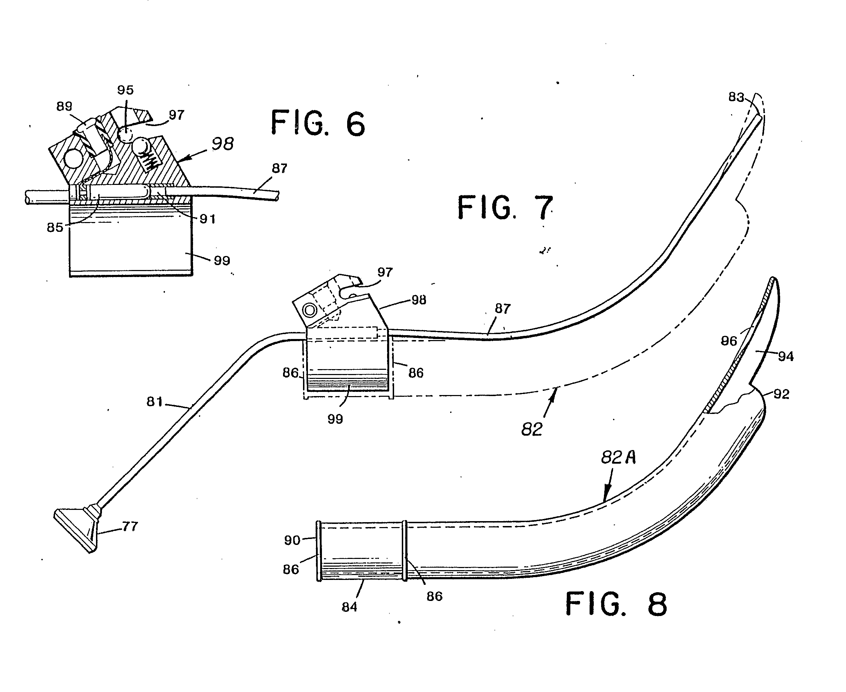

Figs. 6—8 show another embodiment of the laryngoscope blade unit of this invention in which the blade is separable from the base so that the blade can be disposed of after a single use. However, the blade need not be disposable. It can be of metallic material and be washable and capable of being sterilized.

The improved embodiment of Figs. 6—8 relates to a laryngoscope having a blade 82 of substantially the same shape and cross-section as blade 12 of unit 10 except that blade 82 has an end portion 84 provided with a pair of spaced flanges 86 which partially surround blade 82 and have ends which terminate at the slot 88 of the blade, the slot extending throughout a major portion of the blade from end 90 thereof to the opposite end margin 92 near a tip 94 provided with an opening 96.

A base 98 is used with blade 82, and base 98 has a cylindrical, slotted connector section 99 which removably receives end portion 84 of blade 82. Fig. 7 shows the blade in dashed lines in coupled relationship with section 99 with flanges 86 on the end portion 84 of the blade being adjacent to opposed ends of section 96 so as to provide a snug fit for end portion 84 and to prevent axial movement of blade 82 relative to section 99.

Base 98 is of substantially the same construction as base 24 of unit 10 (Fig. 1) except that in this case base 98 is not integral with elongate member 82A. This is shown in more detail in Fig. 6 wherein base 98 has a groove 97 for receiving the cross bar 95 of a handle (not shown) containing a battery, the handle being conventional in construction. A ball detent 93 is spring biased partially across groove 97 to hold bar 95 in place.

Base 98 further has a central bore 91 therethrough for containing a light bulb 85 which serves as a light source for blade unit 80. The bulb is electrically connected to a terminal 89 and to base 98 itself in the manner described above with respect to the electrical connection of bulbs 44 to a battery in a conventional handle. Thus, a description of the electrical connection of the Fig. 1 embodiment suffices for the Fig. 6 embodiment.

A fiber optic light guide 87 extends into a continuation of bore 91 to receive light from bulb 85. Light guide 87 is adapted to be removably inserted at its outer end 83 into hole 96 of blade 82 so that the outer end 83 of the light guide will terminate near tip 94 to direct light forwardly of tip 94 to illuminate the region in advance of the tip.

A fiber optic visual 81 has an eyepiece 77 which extends outwardly from base 98 in the opposite direction from light guide 87. Visual guide 81 has an intermediate part which merges smoothly with light guide 87 and the fibers of visual guide 81 are also embedded in light guide 87 and terminate at end face 83. Commercial fiber optic bundles are available to serve this particular purpose. Thus, light travels from light bulb 85 along light guide 87 to end face 83, whereupon reflected light from an object in advance of end face 83 is received by the fiber optic end face of visual guide 81 and the reflected light travels through visual guide 81 and eyepiece 77. In this way, only a single fiber optics guide is associated with tube 82 to minimize the structure projecting from the outer surface of the blade. While light guide 87 is shown on the back portion of blade 82, it could be at either side as well.

Laryngoscope blade unit 80 is placed in use by first inserting the outer end of light guide 87 through hole 96 of blade 82. Then, end portion 84 of blade 82 is snap-fitted into section 99 of base 98. For this purpose, end portion 84 can be of a semi-rigid or yieldable material, such as a suitable plastic, i.e., polyethelyne or the like. The entire blade, including end portion 84, can be made of this yieldable material: In the alternative, end portion 84 can be of yieldable material and bonded to the remainder of the blade which can be of another material. Instead of a yieldable end portion 84, the latter can be rigid and section 99 on the base 98 can be of a yieldable material.

When base 98 is releasably coupled to the handle, electrical contact is made between the battery and bulb 85, causing the bulb to be activated which illuminates the area in advance of end face 83 of light guide 87. Then, the blade can be inserted into the patient's throat in the manner described above with respect to blade unit 10. Thus, the reflected light, viewed through eyepiece 77, will assist the user of unit 80 in the insertion of the blade into the throat. When the blade is properly inserted, an endotracheal tube is then directed into the open outer end of blade 82 and guided by the blade into and along the blade and eventually into the trachea of the patient. Then, the endotracheal tube and blade 82 can be separated from each other by virtue of the slot 88 in the blade,so that blade 82 can be removed from the throat of the patient leaving the endotracheal tube in place.

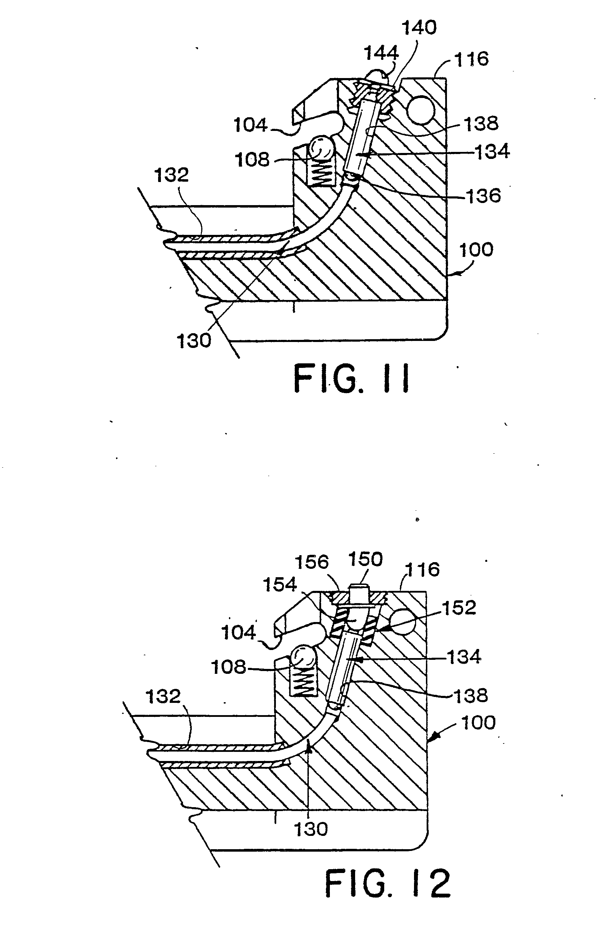

While light bulb 85 is shown centrally of base 98 as indicated in Fig. 11, it is possible that the bulb can be at one side or the other of the base and still be operable. Preferably it is at the center so that light guide 87 can be centrally located relative to blade 82.

Fig. 9 is a view similar to Fig. 1 but showing another embodiment 100 of a base for a laryngoscope blade 102. A slot 104 for receiving the crossbar on a standard handle extends into face 106 of base 100. A ball detent 108 is spring biased into slot 104 for the same reasons as ball 30 of the base of Fig. 1.

A light bulb 110 is received in a cylindrical housing 112 (Fig. 10) in a passage 114 extending into the base from end face 116 thereof. An insulator 118 is press fitted into a second passage 120 and holds housing 112 in place in passage 114. An electrical contact 121 is press fitted into the central bore 122 of insulator 118 and makes electrical contact through a threaded projection 124 extending into a second tubular insulator 126 between the annular extension of contact 121 and threaded end 124. Contact 121 is to be coupled electrically to the corresponding electrical contact on a standard handle when the handle is coupled to base 100 in the usual fashion. This causes bulb 110 to be energized to direct light through a fiber optic bundle 130 (Fig. 9) which extends through base 100 and along a passage 132 in blade 102.

Preferred embodiments of blade base 100 are shown in Figs. 11 and 12 in which another type of housing 134 having a bulb 136 extends in an inclined passage 138 in base 100. An insulator 140 surrounds base screw 144 making contact with the center top pin of bulb 136 and screw 144 has a head defining the end contact for the bulb. This head projects outwardly from end face 116 of base 100. The other electrical connection is through the case of housing 134 and base 100.

Fig. 12 shows that housing 134 can have an electrical contact 150 which project straight out from end face 116 rather than at an angle as shown in Fig. 11. A spacing grommet 152 receives one end 154 of contact 150, the opposite end of the contact being at an angle relative to end 154 as shown in Fig. 12. Contact 150 passes through an insulator 156 threaded into the end of face 116 of base 100. In both embodiments of base 100 as shown in Figs. 11 and 12, fiber optics bundle 130 mates easily with passage 138.

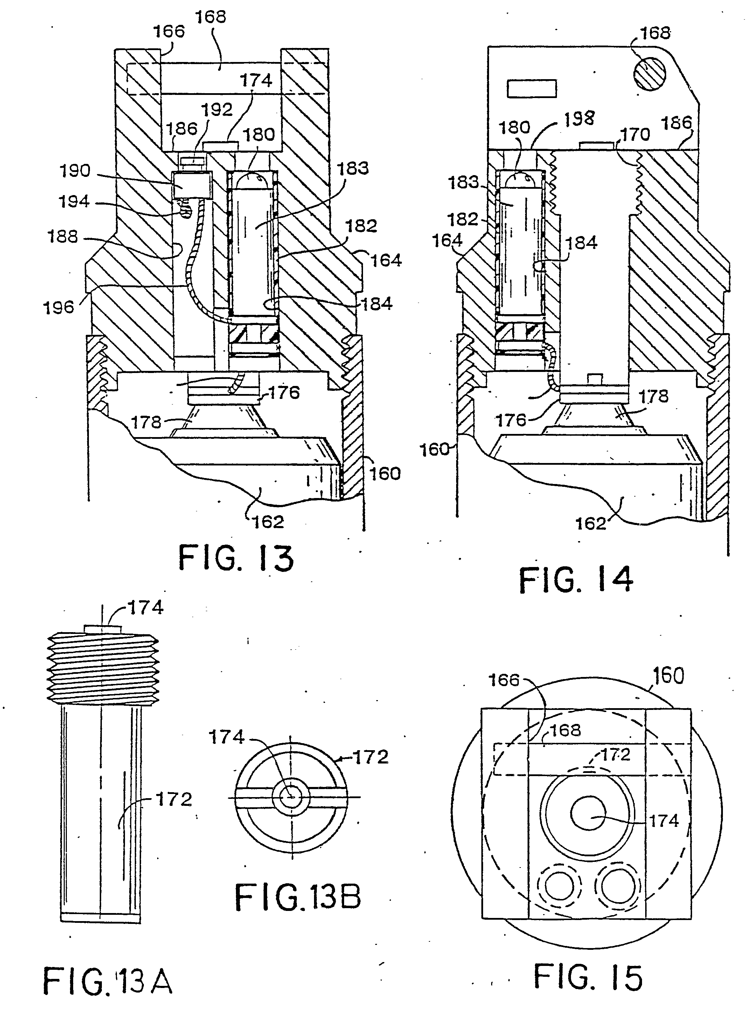

Fig. 13 shows a laryngoscope handle 160 containing a battery 162 and provided with a threaded end member 164 having a slot 166 at the upper end thereof. A crossbar 168 at end 166 is adapted to be inserted into a usual slot, such as slot 104 of base 100 (Fig. 9) for connecting the base to the handle. However, the case of handle 160, member 164 has a space 170 for a cartridge normally found in a standard laryngoscope handle, such as a standard cartridge 172 of the type shown in Figs. 13A and 13B. The cartridge has a contact 174 at its upper end which, through a spring (not shown) inside the housing of the cartridge, makes electrical connection with an end contact 176 which, as shown in Fig. 13, engages the end terminal 178 of battery 162.

Member 164 has a bulb 180 in a cylindrical housing 183 and an insulator 182 disposed within a passage 184 extending inwardly from the end face 186 of member 164. A second passage 188 parallel with passage 184, contains a microswitch 190 having a pushbutton actuator 192 recessed inwardly from end face 186. Also, bulb 180 is spaced inwardly from end face 186. Wires 194 and 196 make electrical connection between battery 162, a light bulb 180 and microswitch 190 when the microswitch is actuated as its actuator 192 is forced inwardly.

Handle 160 is to be used with a conventional laryngoscope blade or a non-conventional blade of the type having a base provided with a fiber optics bundle which is insertable within the opening 198 forming a part of passage 184. The non-conventional blade base also has a projection for insertion into the upper open space above actuator 192 of microswitch 190 so that when the projection is forced into this opening, actuator 192 is depressed and closes switch 190 to connect battery 162 and light bulb 180. Then, light will be transmitted through the fiber optics bundle to the outer end of the laryngoscope blade. If a conventional blade is used, voltage from the battery 162 is supplied to the light bulb at the outer end of the blade through standard cartridge 172 in the usual manner.

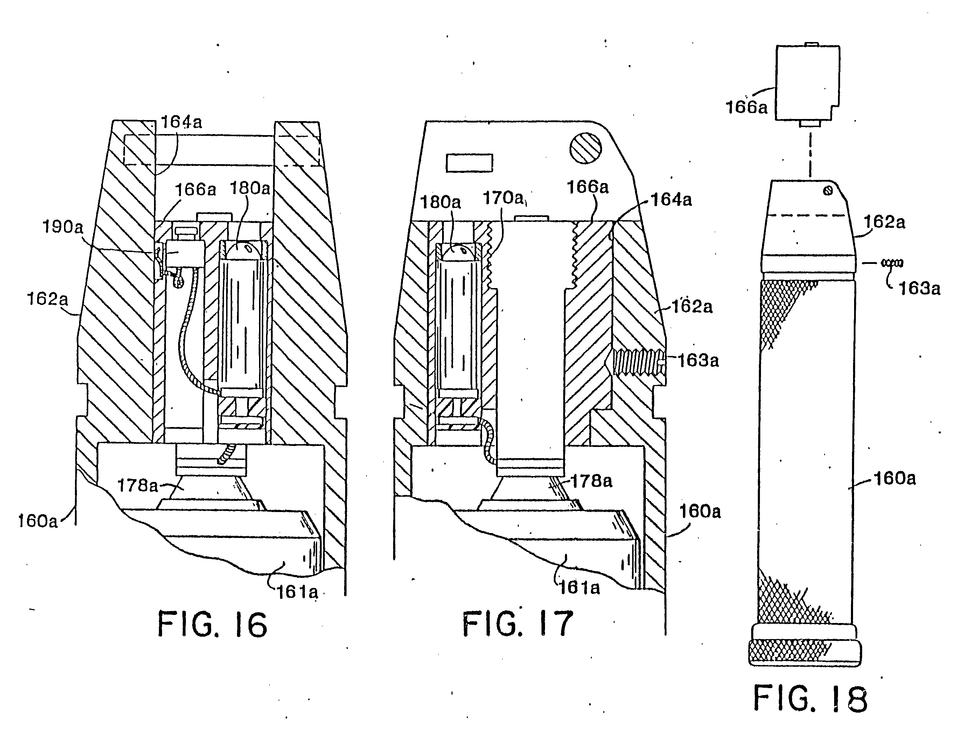

Figs. 16 and 17 are views showing a handle 160a which has an upper member 162a provided with a central, rectangular slot 164a for receiving a cartridge 166 containing a bulb 180a, a standard cartridge 172 of the type shown in Fig. 33 and 34 in a space 170a, and a microswitch 190a. The cartridge is inserted into opening 164a until the cartridge makes electrical contact with the end terminal 178a of a battery 161a. The cartridge is held in place by a set screw 163a threaded into a side opening 165a in member 162a. The cartridge and handle are shown in elevation in Fig. 18.

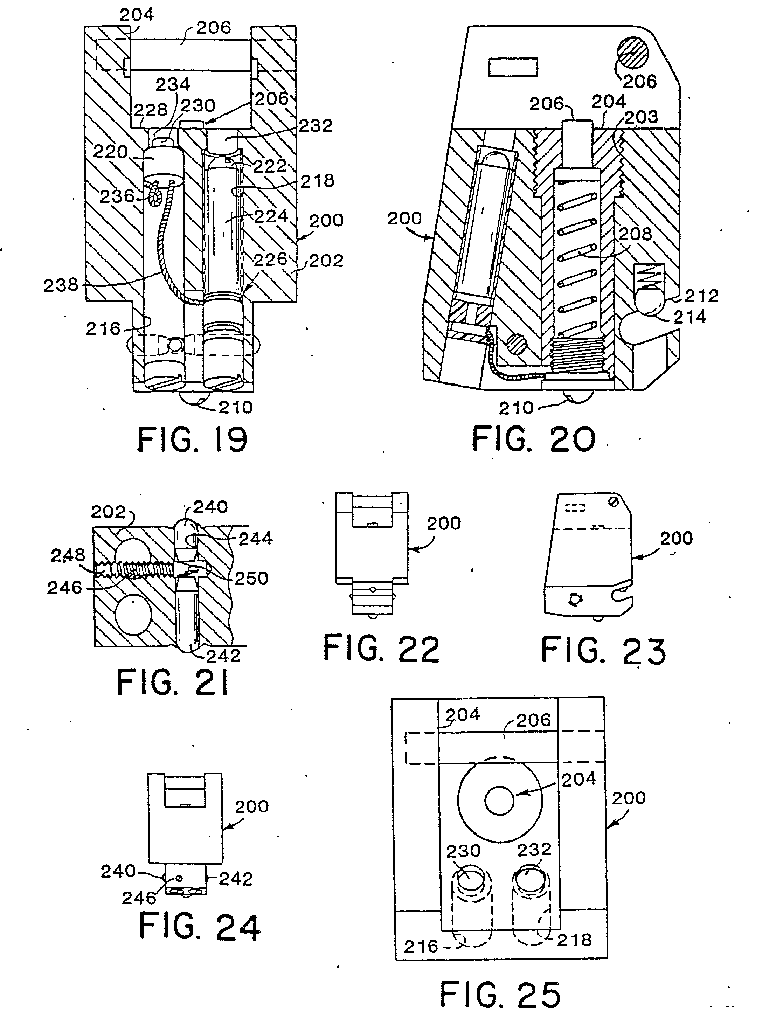

Figs. 19 and 20 show several views of a metallic adapter for use on a handle with a conventional or non-conventional laryngoscope blade. The adapter, denoted by the numeral 200, includes a metallic member 202 having the usual slot 204 provided with a crossbar 206 for attachment to the base of a blade having a slot, such as slot 104 of base 100 (Fig. 9). Member 202 has a central passage 203 for receiving a conventional cartridge 204 of the type having an electrical contact 206 coupled through a metallic, coil spring 208 to an end contact 210 for connection to a battery contact in the conventional handle with which the adapter 200 is to be used. Adapter 200 has a side slot 212 and a ball detent 214 to receive the conventional crossbar of a conventional laryngoscope handle in the usual manner.

Member 202 has two additional, side-by-side, generally parallel passages 216 and 218, passage 216 having a microswitch 220 near the upper end thereof and passage 218 having a light bulb 222 in a housing 224 surrounded by an insulating sleeve 226 inserted into passage 218. The light bulb is recessed inwardly from the upper end surface 228 of member 202. Similarly, the pushbutton actuator 230 of microswitch 220 is also recessed below surface 228. Thus, the recess 232 aligned with the light bulb is adapted to receive the end of the fiber optics bundle on the base of a non-conventional blade which is to be coupled to the upper end of adapter 200. Similarly, the recess 234 aligned with the microswitch is adapted to receive a projection on the non-conventional blade base for actuating the microswitch, which through wires 236 and 238, connects light bulb with the battery in the handle with which adapter 200 is associated. In this way, a light will travel from the light bulb through the fiber optics bundle to the outer end of the blade for illuminating the throat of the patient as the blade is inserted into the throat. If a conventional blade is used with adapter 200, light bulb 222 is not actuated and cartridge 204 will connect the light bulb on the conventional blade with the standard cartridge and battery of the handle.

Figs. 22, 23 and 24 show elevational views of adapter 200. Fig. 25 shows a top plan view of the adapter.

The adapter is held in place by a structure shown in Fig. 21. To this end, a pair of wedges 240 and 242 are mounted in a passage 244 in body 202 as shown in Figs. 19, 20 and 21. A set screw 246 is threaded into a passage 248 perpendicular to passage 244 and has a conical end face 250 which bears against the adjacent ends of wedges 240 and 242 to force the wedges outwardly and against the adjacent surfaces of the conventional handle with which adapter is associated. Fig. 24 also shows screw 246 and the way in which wedges 240 and 242 project laterally from a base portion 252 of adapter 200.

Passages 216 and 218 of adapter 200 are located at an angle so that they can be carried by adapter 200 without interference with cartridge 204. If they were parallel with the cartridge, there would not be sufficient structure to allow for the presence of the light bulb and the microswitch without weakening the overall structure of the adapter.

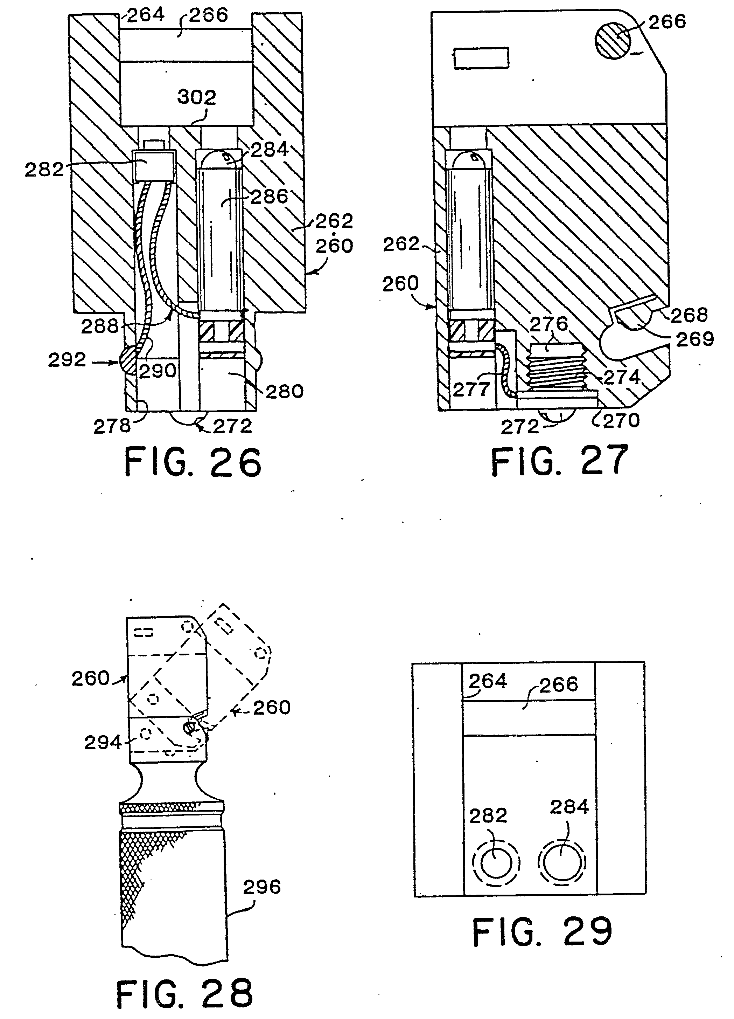

Figs. 26—29 show another form of an adapter which is made of plastic rather than metal as in the case of adapter 200. Adapter 260 has a plastic body 262 provided with an upper slot 264 and a crossbar 266 for attachment to the base of a conventional or non-conventional laryngoscope blade. Body 262 also has a slot 268, a detent 269, and a bottom surface 270 provided with an electrical contact 272 for attachment in the usual fashion with the upper end of a conventional laryngoscope handle. This is shown in Fig. 28, the adapter 260 being shown in an operative position in full lines and in a position ready to be removed from the handle in dashed lines.

Contact 272 has a threaded upper end 274 which is threaded into a passage 276 extending inwardly from surface 270 or body 262. A wire 277 makes electrical contact between a light bulb 284 and contact 272. The adapter has a pair of side-by-side, generally parallel passages 278 and 280 for receiving, respectively, a microswitch 282 and light bulb 284, the latter being carried in a housing 286 and coupled by wire 288 to microswitch 282. A second wire 290 from the microswitch extends to a metal contact 292 for making electrical contact with the adjacent metallic part 294 of conventional handle 296 (Fig. 28). Thus, when adapter 260 is placed on handle 296 in the manner shown in Fig. 28, bulb 284 will be ready to be energized because contact 272 will be coupled to the central terminal of the battery in handle 296 and contact 292 will make electrical contact with part 294 (Fig. 28). When switch 282 is closed, i.e., when the push-button actuator 298 of the switch is pressed downwardly by a projection on a non-conventional blade base coupled with the upper end of the adapter, the light will come on and will pass through a fiber optics bundle whose end is inserted in the recessed opening 300 of passage 280 adjacent to surface 302 of the adapter body 262. Adapter 260 shows that the light source 284 can be accommodated without enlarging the adapter body as in the case of the adapter shown in Figs. 19 and 20.

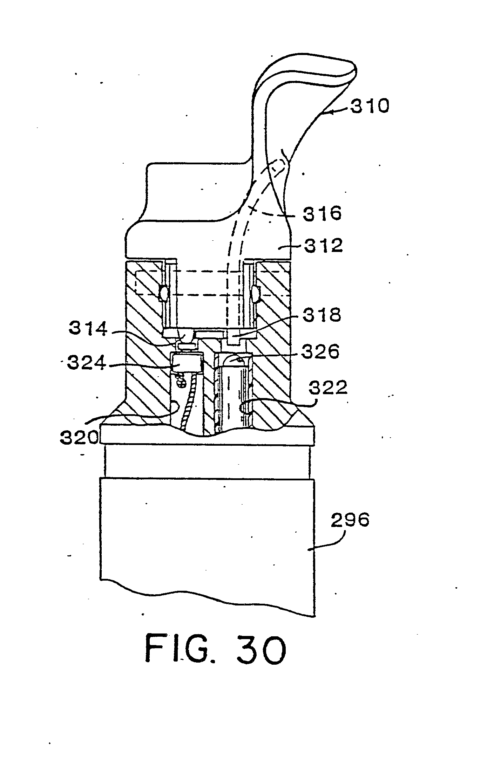

Fig. 30 is a view of a non-conventional laryngoscope blade 310 having a base 312 provided with a projection 314 and a fiber optics bundle 316 having an end 318. Projection 314 and 318 are shown inserted into the end recesses of a pair of passages 320 and 322 containing respectively a microswitch 324 and a light bulb 326. Thus, projection 314 is shown as actuating the microswitch 324 while the fiber optics end 318 is shown aligned optically with the light bulb 326 to receive light therefrom. Fig. 30 illustrates how a base of a blade is used to actuate the light source of adapter 200 of Figs. 19 and 20 and of adapter 260 of Figs. 26 and 27.

## Claims

1. A combination of a laryngoscope and a flexible tube the laryngoscope having a handle (25), and a blade (12; 82) with a base (24; 98 and a tip (20; 94), wherein said blade can be coupled by said base (24) to said handle (25), the blade (12) comprising an elongate member curved for insertion into the throat of a patient and into the pharynx, and the elongate member (12A) being shaped to facilitate the introduction of the flexible tube during intubation, characterised in that said blade (12) is tubular with a passage (12B) for the introduction of said flexible tube; and in that a slot (13) in communication with said passage extends along the full length of said passage, wherein said slot (13) is narrower than said passage (12B) and narrow enough to hold the tube in the passage but wide enough to allow said flexible tube to be separated from the blade (12) when the tube yields so as to become elliptical in shape sufficiently to squeeze or pass through the slot (13).

2. The combination in accordance with claim 1, characterised in that said slot (13) is located at a position within an arc of 90° on each side of the back margin of said blade.

3. The combination in accordance with either of the preceding claims and having a light guide (46; 87) mounted in said elongate member for transmitting light from a light source adjacent said base (24; 98) to a position adjacent said tip (20; 94), characterised in that said light guide (46) comprises a fibre optic guide which extends through a bore (48) in said elongate member (12A).

4. The combination in accordance with claim 3, characterised in that said bore (48) is provided at one side of said elongate member (12A).

5. The combination in accordance with any one of claims 1 to 4, characterised in that a flexible fibre optic viewing guide (74; 81) is provided which extends from an eyepiece (78; 77) movably positioned outside of said blade (12; 82) along said elongate member (12A, 82A) to said tip (20; 94).

6. The combination in accordance with any one of the preceding claims, characterised in that said elongate member (12A) and said base (24) are integral with each other.

7. The combination in accordance with any one of the claims 1 to 5, characterised in that said base (98) and said elongate member (82A) are separable from each other.

8. The combination in accordance with claims 7, characterised in that said base (98) has a tubular connector part (94) which releasably receives one end (84) of the elongate member (82A), the latter being of a yieldable material so that the end (84) of the elongate member (82A) can be snap fitted into place into said connector part (99) of said base (98).

9. The combination in accordance with claim 7 or claim 8, and having a light guide (87) mounted in said elongate member for transmitting light from a light source adjacent said base (98) to a position adjacent said tip (20; 94), characterised in that the light guide (87) is carried by the base (98) and extends outwardly from the base (98) and removably through an opening (96) near the tip (94) of the blade (82A).

10. The combination in accordance with any one of the preceding claims wherein said blade (84) is made of a material which allows the blade (84) to be normally rigid but capable of being flexed or shaped to fit the physiological throat configurations of different patients.

11. The combination in accordance with any one of the preceding claims wherein, for coupling of said blade (12), said handle has a first pair of parallel spaced apart walls defining a first slot and a first cross-bar extending across said first slot, and wherein said handle is adapted to receive a battery power source and has a first electrical contact provided adjacent the base of said first slot, characterised in that an adapter (200) is provided for coupling said blade to said handle, said adapter having a first end connectable with said handle and a second end connectable with said base of said blade; in that said first end has a projection having two spaced side walls adapted to engage in said first slot, a transverse groove (212) adapted to engage with said first cross-bar, and locking means (240, 242, 244, 246, 248, 250) for locking said projection in position in said first slot; in that said second end has a second pair of parallel spaced apart walls defining a second slot and a second cross-bar (206) extending across said second slot; in that said base of said blade has a second projection having two spaced side walls adapted to engage in said second slot and a transverse groove adapted to engage with said second cross-bar (206), there being detent means for releasably securing said base of said blade with said second end of said adapter (200) in an engaged position; in that said handle has a first electrical contact substantially centrally disposed in said said first slot facing a cooperating second contact (210) provided on a base portion of said first projection; in that said second contact (210) is electrically connected to a third contact (206) substantially contrally disposed in said second slot; in that said second contact (210) is electrically connected to a light source (222) provided in said adapter (200); in that a switch is provided in said adapter, said switch (220) being actuatable by said blade in said engaged position to complete an electrical circuit between said light source (222) and said battery power source; in that a second blade having a second light source at its tip can be substituted for the first said blade, said second blade having a fourth contact cooperating with said third contact and a fifth contact cooperating with said second cross-bar (206) to complete an electrical circuit to said battery power source to energise second light source, said second blade being constructed so as not to actuate said switch means; and in that said first and second cross-bars are electrically connected together and to said battery power source when said adapter (200) is locked in position in said first slot.

12. The combination in accordance with claim 11, characterised in that said adapter (200) has a body portion of metal thus ensuring electrical contact between said first and second cross-bars.

13. The combination in accordance with either of the preceding claims 11 and 12, characterised in that said locking means comprises a pair of wedges (240, 242) mounted in a passage (244) in said first projection and a set screw (246) for forcing said wedges (240, 242) outwardly against said first pair of spaced apart walls.

**Patentansprüche**

1. Kombination eines Laryngoskopes und eines flexiblen Rohres, wobei das Laryngoskop einen Handgriff (25) und einen Spatel (12; 82) besitzt mit einer Basis (24; 98) und einer Spitze (20; 94), wobei der Spatel durch die Basis (24) mit dem Handgriff (25) gekoppelt werden Kann, der Spatel (12) ein längliches, zum Einsetzen in die Kehle eines Patienten und in die Pharynx gekrümmtes Teil umfaßt, und das längliche Teil (12A) zur Erleichterung der Einführung des flexiblen Rohres während des Einrohrens geformt ist, dadurch gekennzeichnet, daß der Spatel (12) rohrförmig ist, mit einem Durchlaß (12B) zur Einführung des flexiblen Rohres; und daß ein Schlitz (13) in Verbindung mit dem Durchlaß sich über die volle Länge des Durchlasses erstreckt, wobei der Schlitz (13) enger als der Durchlaß (12B) und eng genug ist, um das Rohr im Durchlaß zu halten, jedoch breit genug, um das Abnehmen des flesiblen Rohres von dem Spatel (12) zuzulassen, wenn das Rohr so weit nachgibt, daß es ausreichend elliptisch geformt wird, um sich durch den Schlitz (13) hindurchzuquetschen oder durchzugehen.

2. Kombination nach Anspruch 1, dadurch gekennzeichnet, daß der Schlitz (13) an einer Stelle innerhalb eines Bogens von 90° zu beiden Seiten der hinteren Begrenzung des Spatels angeordnet ist.

3. Kombination nach einem der vorangehenden Ansprüche und mit einem in dem länglichen Teil angebrachten Lichtleiter (46; 87) zum Übertragen von Licht von einer zur Basis (24; 98) benachbarten Lichtquelle zu einer der Spitze (20; 94) benachbarten Stelle, dadurch gekennzeichnet, daß der Lichtleiter (46) einen Faseroptik-Leiter umfaßt, der sich durch eine Bohrung (48) in dem länglichen Teil (12A) erstreckt.

4. Kombination nach Anspruch 3, dadurch gekennzeichnet, daß die Bohrung (48) an einer Seite des länglichen Teiles (12A) vorgesehen ist.

5. Kombination nach einem der Ansprüche 1 bis 4, dadurch gekennzeichnet, daß ein flexibler Faseroptik-Sichtleiter (74; 81) vorgesehen ist, der sich von einem beweglich außerhalb des Spatels (12; 82) angeordneten Okkular (78; 77) längs des länglichen Teiles (12A; 82A) zu der Spitze (20; 94) erstreckt

6. Kombination nach einem der vorangehenden Ansprüche, dadurch gekennzeichnet, daß das längliche Teil (12A) und die Basis (24) integral miteinander sind.

7. Kombination nach einem der Ansprüche 1 bis 5, dadurch gekennzeichnet, daß die Basis (98) und das längliche Teil (82A) voneinander abtrennbar sind.

8. Kombination nach Anspruch 7, dadurch gekennzeichnet, daß die Basis (98) ein rohrförmiges Verbindungsteil (94) besitzt, das lösbar ein Ende (84) des länglichen Teiles (82A) aufnimmt, daß das letztere aus einem nachgiebigen Material besteht, so daß das Ende (84) des länglichen Teiles (82A) durch Einschnappen an seinen Ort in den Verbindungsteil (99) der Basis (98) eingepaßt werden kann.

9. Kombination nach Anspruch 7 oder 8, und mit einem in dem länglichen Teil zur Übertragung von Licht von einer der Basis (98) benachbarten Lichtquelle zu einer der Spitze (20; 94) benachbarten Stelle angebrachten Lichtleiter (87), dadurch gekennzeichnet, daß der Lichtleiter (87) durch die Basis (98) gehalten ist und sich von der Basis (98) nach außen und entfernbar durch eine Öffnung (96) in der Nähe der Spitze (94) des Spatels (82A) erstreckt.

10. Kombination nach einem der vorangehenden Ansprüche, bei der der Spatel (84) aus einem Material gefertigt ist, das zuläßt, daß der Spatel (84) normalerweise starr, jedoch zu einem Biegen oder Formen zum Anpassen an die physiologischen Kehlenformen verschiedener Patienten fähig ist.

11. Kombination nach einem der vorangehenden Ansprüche, bei der zum Koppeln des Spatels (12) der Handgriff ein erstes Paar parallel mit Abstand versehener, einen ersten Schlutz bestimmende Wände und einen ersten, sich über den Schlitz erstreckenden Querstab besitzt, und bei der der Handgriff zur Aufnahme einer Batterie-Energiequelle ausgelegt ist und einen ersten, der Basis des ersten Schlitzes benachbart vorgesehenen elektrischen Kontakt besitzt, dadurch gekennzeichnet, daß ein Adapter (200) zum koppeln des Spatels mit dem Handgriff vorgesehen ist, wobei der Adapter ein erstes mit dem Handgriff verbindbares Ende und ein zweites, mit der Basis des Spatels verbindbares Ende besitz, daß das erste Ende einen Fortsatz mit zwei mit Abstand voneinander versehenen Seitenwänden, zum Eingriff in den ersten Schlitz, eine zum Eingriff mit dem ersten Querstab ausgelegte Quernut (212) und Sperrmittel (240, 242, 244, 246, 248, 250) zum Versperren des Fortsatzes in seiner Stellung in dem ersten Schlitz besitzt, daß das zweite Ende ein zweites Paar von einen zweiten Schlitz bestimmenden, parallelen, mit Abstand voneinander versehenen Wänden und einen sich über den zweiten Schlitz erstreckenden zweiten Querstab (206) besitzt; daß die Basis des Spatels einen zweiten Fortsatz besitzt mit zwei mit Abstand voneinander versehenen Seitenwänden, zum Eingriff in den zweiten Schlitz geeignet, und eine zum Eingriff mit dem zweiten Querstab (206) ausgelegte Quernut, wobei Rastmittel zum lösbaren Sichern der Basis des Spatels an dem zweiten Ende des Adapters (200) in einer Eingriffsstellung vorhanden sind; daß der Handgriff einen im wesentlichen zentral in dem ersten Schlitz angeordneten ersten elektrischen Kontakt besitzt, der einem damit zusammenwirkenden, an einem Grundabschnitt des ersten Fortsatzes vorgesehenen zweiten Kontakt (210) zugewendet ist; daß der zweite Kontakt (210) elektrisch mit einem im wesentlichen in dem zweiten Schlitz zentral angeordneten dritten Kontakt (206) verbunden ist; daß der zweite Kontakt (210) mit einer in dem Adapter (200) vorgesehenen Lichtquelle (222) elektrisch verbunden ist, wobei der Schalter (220) durch den Spatel in der Eingriffsstellung zur Herstellung eines elektrischen Kreises zwischen der Lichtquelle (222) und der Batterie-Energiequelle betätigbar ist; daß ein zweiter Spatel mit einer Lichtquelle an seiner Spitze statt des ersten Spatels einsetzbar ist, wobei der zweite Spatel einen mit dem dritten Kontakt zusammenwirkenden vierten Kontakt und einen mit dem zweiten Querstab (206) zusammenwirkenden fünften Kontakt zur Herstellung eines elektrischen Kreises zu der Batterie-Energiequelle zur Beaufschlagung der zweiten Lichtquelle besitzt, wobei der zweite Spatel so aufgebaut ist, daß er nicht das Schaltmittel betätigt; und daß der erste und der zweite Querstab miteinander und mit der Batterie-Energiequelle elektrisch verbunden sind, wenn der Adapter (200) in seiner Stellung im ersten Schlitz versperrt ist.

12. Kombination nach Anspruch 11, dadurch gekennzeichnet, daß der Adapter (200) einen Gehäuseabschnitt aus Metall besitzt, um so elektrische Verbindung zwischen dem ersten und dem zweiten Querstab sicherzustellen.

13. Kombination nach einem der vorangehenden Ansprüche 11 und 12, dadurch gekennzeichnet, daß die Sperrmittel ein Paar in einem Durchlaß (244) in dem ersten Fortsatz angebrachte Keile (240, 242) und eine Stellschraube (246) umfaßt zum Drücken der Keile (240, 242) nach außen gegen das erste Paar gegeneinander mit Abstand versehener Wände.

**Revendications**

1. Combinaison d'un laryngoscope et d'un tube flexible, le laryngoscope comportant une poignée (25), et une lame (12; 82) avec une embase (24;

98) et une pointe (20; 94), ladite lame pouvant être accouplée par ladite embase (24) à ladite poignée (25), la lame (12) comprenant un élément allongé incurvé pour être inséré dans la gorge d'un patient et dans le pharynx, et l'élément allongé (12A) étant configuré de façon à faciliter l'introduction du tube flexible pendant une intubation, caractérisé en ce que ladite lame (12) est tubulaire et présente un passage (12B) pour l'introduction dudit tube flexible; et en ce qu'une fente (13) en communication avec ledit passage s'étend sur toute la longueur dudit passage, ladite fente (13) étant plus étroite que ledit passage (12B) et suffisamment étroite pour maintenir le tube dans le passage, mais suffisamment large pour permettre audit tube flexible d'être séparé de la lame (12) lorsque le tube fléchit de façon à prendre une forme elliptique suffisante pour être comprimé ou passé à travers la fente (13).

2. Combinaison selon la revendication 1, caractérisée en ce que ladite fente (13) est située dans une position à l'intérieur d'un arc de 90° de chaque côté du bord arrière de ladite lame.

3. Combinaison selon l'une des revendications précédentes, et ayant un guide (46; 87) de lumière monté dans ledit élément allongé pour transmettre la lumière provenant d'une source de lumière adjacente à ladite embase (24; 98) jusqu'à une position adjacente à ladite pointe (20; 94), caractérisée en ce que ledit guide (46) de lumière comprend un guide à fibre optique qui passe dans un alésage (48) dudit élément allongé (12A).

4. Combinaison selon la revendication 3, caractérisée en ce que ledit alésage (48) est prévu sur un premier côté dudit élément allongé (12A).

5. Combinaison selon l'une quelconque des revendications 1 à 4, caractérisée en ce qu'un guide flexible (74; 81) de visée à fibre optique est prévu et s'étend d'un oculaire (78; 77), monté de façon mobile à l'extérieur de ladite lame (12; 82), le long dudit élément allongé (12A, 82A), jusqu'à ladite pointe (20; 94).

6. Combinaison selon l'une quelconque des revendications précédentes, caractérisée en ce que ledit élément allongé (12A) et ladite embase (24) sont réalisés d'une seule pièce l'un avec l'autre.

7. Combinaison selon l'une quelconque des revendications 1 à 5, caractérisée en ce que ladite embase (98) et ledit élément allongé (82A) peuvent être séparés l'un de l'autre.

8. Combinaison selon la revendication 7, caractérisée en ce que ladite embase (98) comporte une partie de raccord tubulaire (94) qui reçoit de manière amovible une première extrémité (84) de l'élément allongé (82A), ce dernier étant en une matière flexible afin que l'extrémité (84) de l'élément allongé (82A) puisse être enclenché élastiquement en position dans ladite partie (99) de raccord de ladite embase (98).

9. Combinaison selon la revendication 7 ou la revendication 8, et comportant un guide (87) de lumière monté dans ledit élément allongé afin de transmettre la lumière provenant d'une source de lumière adjacente à ladite embase (98) jusqu'à une position adjacente à ladite pointe (20; 94), caractérisée en ce que le guide (87) de lumière est porté par l'embase (98) et s'étend vers l'extérieur de l'embase (98) et de manière amovible à travers une ouverture (96) proche de la pointe (94) de la lame (82A).

10. Combinaison selon l'une quelconque des revendications précédentes, dans laquelle ladite lame (84) est réalisée en une matière qui permet à la lame (84) d'être normalement rigide, mais de pouvoir être courbée ou façonnée pour s'adapter aux configurations physiologiques des gorges de différents patients.

11. Combinaison selon l'une quelconque des revendications précédentes, dans laquelle, pour l'accouplement de ladite lame (12), ladite poignée comporte une première paire de parois parallèles et espacées définissant une première rainure et une première tige transversale s'étendant en travers de ladite première rainure, et dans laquelle ladite poignée est conçue pour recevoir une source d'énergie à batterie et comporte un premier contact électrique prévu à proximité immédiate de la base de ladite première rainure, caractérisée en ce qu'un adaptateur (200) est prévu pour accoupler ladite lame à ladite poignée, ladit adaptateur présentant une première extrémité pouvant être raccordée à ladite poignée et une seconde extrémiteré pouvant être raccordée à ladite embase de ladite lame; en ce que ladite première extrémité comporte une saillie présentant deux parois latérales espacées conçues pour s'engager dans ladite première rainure, une gorge transversale (212) cónçue pour s'enclencher avec ladite première tige transversale et des moyens de verrouillage (240, 242, 244, 246, 248, 250) destinés à verrouiller ladite saillie en position dans ladite première rainure; en ce que ladite seconde extrémité comporte une seconde paire de parois parallèles et espacées définissant une seconde rainure et une seconde tige transversale (206) s'étendant en travers de ladite seconde rainure; en ce que ladite embase de ladite lame comporte une seconde saillie présentant deux parois latérales espacés conçues pour s'engager dans ladite seconde rainure et une gorge transversale conçue pour s'enclencher avec ladite seconde tige transversale (206), des moyens d'encliquetage étant destinée à fixer de façon amovible ladite embase de ladite lame à ladite seconde extrémité dudit adaptateur (200) dans une position enclenchée; en ce que ladite poignée comporte un premier contact électrique disposé à peu près centralement dans ladite première rainure, face à un second contact électrique coopérant (210) prévu sur une partie de base de ladite première saillie; en ce que ledit deuxième contact (210) est connecté électriquement à un troisième contact (206) disposé à peu près centralement dans ladite seconde rainure; en ce que ledit deuxième contact (210) est connecté électriquement à une source (222) de lumière prévue ledit adaptateur (200), ledit commutateur (220) pouvant être actionné par ladite lame dans ladite position d'enclenchement afin de fermer un circuit électrique entre ladite source de lumière (222) et ladite source d'énergie à batterie; en ce qu'une seconde lame comportant une seconde source de lumière à sa pointe peut être substituée à la première lame, ladite seconde lame comportant un quatrième contact coopérant avec ledit troisième contact et un cinquième contact coopérant avec ladite seconde tige transversale (206) pour fermer un circuit électrique vers ladite source d'énergie à batterie afin d'alimenter ladite seconde source de lumière, ladite seconde lame étant réalisée de façon à ne pas actionner ledit commutateur; et en ce que lesdites première et seconde tiges transversales sont connectées électriquement entre elles et à ladite source d'énergie à batterie lorsque ledit adaptateur (200) est verrouillé en position dans ladite première rainure.

12. Combinaison selon la revendication 11, caractérisée en ce que ledit adaptateur (200) comporte une partie de corps en métal, assurant ainsi un contact électrique entre lesdites première et seconde tiges transversales.

13. Combinaison selon l'une des revendications précédentes 11 et 12, caractérisée en ce que lesdits moyens de verrouillage comprennent deux coins (240, 242) montés dans un passage (244) situé dans ladite première saillie, et une vis (246) de blocage destinée à appliquer à force vers l'extérieur lesdits coins (240, 242) contre ladite première paire de parois espacées.

66 38 28 10 FIG. 1
68 36
72 30
24 32 26
70 34 76 74
74
64
62 48
58 46
60 56
52
54 44 40

FIG. 2
25
20
5
50
24
76 12
74
22
40 46
78

# FIG. 3

# FIG. 5

# FIG. 4

FIG. 6

FIG. 7

FIG. 8

FIG. 9

FIG. 10

FIG. II

FIG. 12

FIG. 13

FIG. 14

FIG.13B

FIG.13A

FIG. 15

6

FIG. 16

FIG. 17

FIG. 18

0 030 014

FIG. 19

FIG. 20

FIG. 21

FIG. 22

FIG. 23

FIG. 24

FIG. 25

FIG. 26

FIG. 27

FIG. 28

FIG. 29

FIG. 30